# EUROPÄISCHE PATENTSCHRIFT

(11) **EP 2 664 278 B1**
(45) Veröffentlichungstag und Bekanntmachung des Hinweises auf die Patenterteilung: **10.06.2015**
(21) Anmeldenummer: 13176528.1
(22) Anmeldetag: 12.06.2010
(51) Int. Cl.: A61B 5/151

(54) **Lanzette**
Lance
Lancette

(30) Priorität: 10.07.2009 EP 09009000
(43) Veröffentlichungstag der Anmeldung: 20.11.2013
(62) Teilanmeldung aus: 10725394.0
(73) Patentinhaber: Roche Diagnostics GmbH, 68305 Mannheim (DE); F. Hoffmann-La Roche AG, 4070 Basel (CH)
(72) Erfinder: Hörauf, Christian, 68723 Oftersheim (DE)
(74) Vertreter: Twelmeier Mommer & Partner

(56) Entgegenhaltungen:
- EP-A- 1 709 906
- EP-A- 2 022 399
- EP-A1- 1 759 633
- EP-A2- 1 346 686
- WO-A-2005/084545

## Beschreibung

Die Erfindung geht aus von einer Lanzette mit den im Oberbegriff des Anspruchs 1 angegebenen Merkmalen. Eine derartige Lanzette ist aus der WO 2005/084545 A1 bekannt.

Aus der EP 1 759 633 A1 ist eine Lanzette bekannt, die aus schräg angeschliffenem Draht hergestellt ist. Durch das schräge Anschleifen des Drahtes wird eine Schlifffläche geschaffen, die beidseitig von Kanten begrenzt ist, die in einer Lanzenspitze zusammen laufen. In der Schlifffläche ist ein beidseitig offener Längsschlitz zur Probenaufnahme angeordnet.

Ständiges Ziel bei der Entwicklung von Lanzetten ist es, eine möglichst schmerzarme Probenentnahme zu ermöglichen. Aufgabe der vorliegenden Erfindung ist es, einen Weg aufzuzeigen, wie dieses Ziel noch besser erreicht werden kann.

Diese Aufgabe wird durch eine Lanzette mit den im Anspruch 1 angegebenen Merkmalen gelöst. Vorteilhafte Weiterbildungen der Erfindung sind Gegenstand von Unteransprüchen.

Eine erfindungsgemäße Lanzette hat einen flachen Schaft, der eine Oberseite und eine Unterseite aufweist, die über sich in Längsrichtung des Schafts erstreckende Schmalseiten miteinander verbunden sind. Ein solcher Schaft kann beispielsweise aus Blech mit geringem Aufwand hergestellt werden. Der Schaft einer erfindungsgemäßen Lanzette bildet an einem vorderen Ende eine Schneide, die in einer Spitze endet. Die Schneide weist an der Unterseite zwei Schneidkanten auf, die in der Spitze zusammenlaufen. Auf seiner Unterseite weist der Schaft wenigstens eine Vertiefung zur Probenaufnahme auf. Diese Vertiefung ist als eine Rinne oder Rille ausgebildet.

Bei einer erfindungsgemäßen Lanzette ist die Schneide an der Oberseite durch zwei Kanten begrenzt, die in einem Scheitelpunkt zusammenlaufen. Die Oberseite der Schneide ist bei einer vorteilhaften Weiterbildung der Erfindung zwischen dem Scheitelpunkt und dem vorderen Ende im Querschnitt gesehen konvex gewölbt. Zwischen dem Scheitelpunkt und dem vorderen Ende hat die Schneide dann also quer zur Längsrichtung gesehen eine konvex gerundete Oberseite. Diese besondere Form der Schneide ermöglicht einen schmerzärmeren Einstich, als dies mit den aus der WO 2005/084545 A1 bekannten Lanzetten möglich ist, deren Schneiden von ebenen Flächen und einer einzigen oberen Kante, die senkrecht zur Längsrichtung des Schafts verläuft, begrenzt sind.

Die überraschend vorteilhaften Eigenschaften einer Lanzette gemäß der vorteilhaften Weiterbildung der Erfindung können wahrscheinlich darauf zurückgeführt werden, dass sich bei einem Einstich die Ausbildung schmerzhafter Druckwellen weitestgehend vermeiden lässt. Beim Eindringen einer erfindungsgemäßen Lanzette in Körpergewebe eines Patienten wird ein Einstichkanal erzeugt, der durch die konvex gewölbte Oberseite der Schneide schonend auf die Dicke des Lanzettenschafts gedehnt wird. Da die Schneide an der Oberseite durch zwei zusammenlaufende Kanten begrenzt ist und zwischen dem Scheitelpunkt und dem vorderen Ende die Oberseite der Schneide im Querschnitt gesehen konvex gewölbt ist, wird der Einstichkanal zunächst nur auf einem Teil seiner Breite auf die volle Dicke des Lanzettenschafts gedehnt. Bei dem weiteren Eindringen der Lanzette wird der Einstichkanal dann auf einem zunehmend größeren Teil seiner Breite auf die volle Dicke des Lanzettenschafts aufgeweitet. Dies ist eine wesentliche Verbesserung gegenüber der aus der WO 2005/084545 A1 bekannten Lanzette, bei welcher der Einstichkanal bei Erreichen der vollen Dicke gleichzeitig auch auf die volle Breite gedehnt wird.

Wegen der endlichen Dicke und Breite einer Lanzette ist es unvermeidbar, dass bei einem Stich auch quer zur Stichrichtung Druck auf umgebendes Gewebe ausgeübt wird. Vorteilhaft wird dieser Druck bei einer Lanzette gemäß der vorteilhaften Weiterbildung der Erfindung praktisch ausschließlich von der gewölbten Oberseite der Schneide und der daran anschließenden Oberseite des Schafts ausgeübt. Dies führt wohl dazu, dass an der Unterseite des Schafts anliegendes Gewebe weniger stark komprimiert wird und deshalb Körperflüssigkeit aus dem an der Unterseite des Schafts anliegenden Gewebe besonders leicht austreten und die zur Probenaufnahme an der Unterseite des Schafts vorgesehene Vertiefung füllen kann.

Eine weitere vorteilhafte Weiterbildung der Erfindung sieht vor, dass die Schneide entlang einer von dem Scheitelpunkt zu dem vorderen Ende verlaufenden Linie konkav geformt ist. Diese Maßnahme trägt dazu bei, den mit einem Einstich verbundenen Schmerz weiter zu reduzieren. Auf diese Weise nimmt nämlich die Dicke der Schneide von der Spitze aus gesehen zunächst nur relativ langsam und später stärker zu. Die Dicke der Schneide ist deshalb in einen vorderen Bereich reduziert. Es wird vermutet, dass Körpergewebe bei einem Einstich der Lanzette zunächst einen höheren Widerstand entgegensetzt, der beim Vordringen der Lanzette abnimmt. Mit einer erfindungsgemäß geformten Schneide ist die zum Eindringen einer Lanzette in Körpergeweben erforderliche Kraft und damit auch das Schmerzempfinden vorteilhaft reduziert. Die Schneide einer erfindungsgemäßen Lanzette ist bevorzugt in Längsrichtung konkav und in Querrichtung konvex gerundet.

Der Scheitelpunkt liegt bevorzugt ebenso wie die Spitze in der Mitte der Breite des Schafts. Es ist aber auch möglich, den Scheitelpunkt und/oder die Spitze versetzt von der Mitte anzuordnen, so dass die beiden Schneidkanten bzw. die beiden Oberkanten der Schneide unterschiedliche Längen aufweisen. In einem solchen Fall verläuft die Linie von dem Scheitelpunkt zu dem vorderen Ende nicht exakt in Längsrichtung des Schafts, sondern leicht schräg dazu.

Eine weitere vorteilhafte Weiterbildung der Erfindung sieht vor, dass sich die oberen Kanten der Schneide weiter nach hinten erstrecken als die Schneidkanten. Auf diese Weise kann der mit einem Einstich verbundene Schmerz weiter reduziert werden. Die Schneide ist dabei bevorzugt durch seitliche Kanten begrenzt, welche das hintere Ende einer Schneidkante mit dem hinteren Ende einer der oberen Kanten verbinden. Die seitlichen Kanten schließen bevorzugt mit einer hinter der Schneide verlaufenden Unterkante des Schafts einen spitzen Winkel ein, der beispielsweise 10° bis 60°, insbesondere zwischen 15° und 35°, betragen kann.

Eine weitere vorteilhafte Weiterbildung der Erfindung sieht vor, dass die zur Probenaufnahme vorgesehene Vertiefung als eine Rinne ausgebildet ist. Bevorzugt endet diese Rinne in einem Abstand von dem vorderen Ende des Schafts, insbesondere zwischen dem Scheitelpunkt und dem vorderen Ende des Schafts. Besonders bevorzugt hat die Rinne einen zwischen den Schneidkanten angeordneten Abschnitt, in welchem die Querschnittsfläche der Rinne zu dem vorderen Ende hin abnimmt. Die Querschnittsfläche der Rinne kann im Bereich der Schneide abnehmen, indem sich die Breite oder die Tiefe der Rinne, bevorzugt sowohl die Breite als auch die Tiefe, reduziert. Bevorzugt nimmt die Querschnittsfläche, also die Breite und/oder die Tiefe, auf einer Länge ab, die größer als die maximale Breite der Rinne ist. Auf diese Weise kann die Rinne näher an das vordere Ende der Schneide heranreichen, ohne dass die mechanische Stabilität der Schneide beeinträchtigt wird.

Bevorzugt hat die Schneide am Ende der Rinne weniger als zwei Drittel, vorzugsweise nicht mehr als die Hälfte, der Dicke des Schafts. Besonders bevorzugt hat die Schneide am Ende der Rinne weniger als zwei Drittel, vorzugsweise nicht mehr als die Hälfte, der Breite des Schafts. Hinter der Schneide beträgt die Tiefe der Rinne bevorzugt mehr als die Hälfte der Schaftdicke.

Für einen Flüssigkeitstransport durch Kapillarkräfte ist an sich erforderlich, dass sich der Kapillarquerschnitt nicht vergrößert, da ein Vordringen von Flüssigkeit in eine sich vergrößernde Kapillare eine Vergrößerung der Grenzfläche zwischen Flüssigkeit und Luft bedeutet und deshalb energetisch ungünstig ist. Man könnte deshalb vermuten, dass ein sich verjüngender Abschnitt der Rinne zur Probenaufnahme nichts beitragen kann. Überraschender Weise ist dies jedoch nicht der Fall. Indem die Rinne eine zu dem Ende der Schneide hin abnehmende Querschnittfläche hat, kann die für eine Probenaufnahme erforderliche Stichtiefe reduziert werden. Vorteilhaft ist deshalb mit einer erfindungsgemäßen Lanzette eine Probenaufnahme mit weniger Schmerz verbunden.

Der Aspekt der Erfindung, an der Unterseite der Lanzette eine Rinne vorzusehen, deren Querschnittsfläche zu dem vorderen Ende hin abnimmt, hat deshalb auch eigenständige Bedeutung. Die vorliegende Erfindung betrifft deshalb auch eine Lanzette mit einem flachen Schaft, der eine Oberseite und eine Unterseite hat, wobei der Schaft an einem vorderen Ende eine Schneide ausbildet, die in einer Spitze endet, die Schneide an der Unterseite zwei Schneidkanten aufweist, die in der Spitze zusammenlaufen, und der Schaft auf seiner Unterseite wenigstens eine Rinne zur Probenaufnahme aufweist, wobei die Rinne einen zwischen den Schneidkanten angeordneten Abschnitt aufweist, in welchem die Querschnittsfläche der Rinne zu dem vorderen Ende hin abnimmt. Bevorzugt nimmt die Querschnittsfläche auf einer Länge ab, die größer als die Rinnenbreite ist, besonders bevorzugt größer als die Schaftdicke, insbesondere größer als die Schaftbreite.

Ein Vorteil einer erfindungsgemäßen Lanzette mit einer an der Unterseite des Schafts angeordneten Rinne zur Probenaufnahme besteht insbesondere auch darin, dass die Gefahr einer Vorstopfung der Rinne oder einer Beeinträchtigung einer in der Rinne enthaltenen hydrophilen Beschichtung reduziert ist.

Eine weitere vorteilhafte Weiterbildung der Erfindung sieht vor, dass die beiden Kanten, welche die Schneide an der Oberseite begrenzen, parallel zu den Schneidkanten verlaufen.

Eine weitere vorteilhafte Weiterbildung der Erfindung sieht vor, dass der Scheitelpunkt um mehr als die Dicke des Schaftes hinter der Spitze angeordnet ist.

Eine weitere vorteilhafte Weiterbildung der Erfindung sieht vor, dass die beiden Kanten, welche die Schneide an der Oberseite begrenzen, einen spitzen Winkel einschließen.

Eine weitere vorteilhafte Weiterbildung der Erfindung sieht vor, dass die Unterseite der Schneide zumindest in einem entlang der Schneidkanten verlaufenden Randbereich plan ist.

Eine erfindungsgemäße Lanzette wird bevorzugt aus Metall, bevorzugt aus Stahl hergestellt. Als Lanzettenschaft wird dabei ein Streifen Metallblech verwendet. Durch Ätzen, bevorzugt nasschemisches Metallätzen, können die Schneide und eine oder mehrere Vertiefungen zur Probenaufnahme geformt werden. Dazu kann ein Streifen Metallblech allseitig mit einem Fotolack beschichtet werden. Durch geeignetes Belichten und anschließendes Abwaschen kann der Fotolack an der Unterseite dort entfernt werden, wo die wenigstens eine Vertiefung zur Probenaufnahme ausgebildet werden soll. An der Oberseite kann der Fotolack im gesamten Bereich vor zwei V-förmigen Linien, die später die oberen Kanten der Schneide bilden, entfernt werden. Zusätzlich kann der Fotolack an den Schmalseiten im Bereich vor einer von oben nach unten verlaufenden Linie, welche später eine seitliche Begrenzungskante der Schneide bildet, entfernt werden. Durch anschließendes Einwirken eines Ätzmediums kann dann die erfindungsgemäße Form der Schneide hergestellt werden. Alternativ kann eine erfindungsgemäße Lanzette aber beispielsweise mittels Laserstrahlschneiden hergestellt werden.

Vorteilhaft kann mit einer erfindungsgemäßen Lanzette eine Probe unter der Haut aufgenommen werden. Die Gefahr einer Verunreinigung an der Hautoberfläche kann somit vermieden werden. Dies ist insbesondere bei der Glucosekonzentrationsbestimmung ein wichtiger Vorteil, da Zucker auf der Haut, beispielsweise nach dem Verzehr von Süßspeisen, häufig vorkommt. Um eine Probe unter der Haut aufnehmen zu können, können vorteilhaft Stechgeräte verwendet werden, bei denen die Rückführbewegung der Lanzette langsamer als die Vorschubbewegung erfolgt. Geräte mit geeigneten Stechantrieben sind in der EP 1 709 906 A1 und der US 2008/0262388 A1 beschrieben.

Ein Aspekt der vorliegenden Erfindung betrifft deshalb ein Stechsystem mit einer erfindungsgemäßen Lanzette und einem Stechgerät, das bei einem Stich eine Vorschubbewegung der Lanzette und eine daran anschließende Rückführbewegung bewirkt, wobei die Rückführbewegung langsamer als die Vorschubbewegung ist. Bevorzugt wird die Lanzette bei einem ersten Abschnitt der Rückführbewegung schneller als in einem daran anschließenden zweiten Abschnitt zurückgezogen. Auf diese Weise bleibt die Lanzette nur eine möglichst kurze Zeit in schmerzempfindlichem Körgewebe, verweilt jedoch für eine Probenaufnahme längere Zeit in schmerzunempfindlichem Körpergewebe, beispielsweise dem Stratum corneum.

Eine erfindungsgemäße Lanzette kann so ausgebildet sein, dass ihre Schneide bei einem Einstich durch Körpergewebe quer zur Stichrichtung abgelenkt und zu ihrer Unterseite hin gebogen wird. Bei der Rückführbewegung entsteht dann zwischen der Unterseite der Schneide und dem Gewebe ein Hohlraum, der sich rasch mit Körperflüssigkeit füllt. Während der langsamen Rückführbewegung, bzw. während des langsamen Abschnitts der Rückführbewegung kann die an der Unterseite der Lanzette angeordnete Vertiefung vorteilhaft eine Probe aufnehmen. Das Entstehen eines Hohlraums zwischen der Unterseite der Schneide und umgebendem Körpergewebe kann auch durch eine beim Einstich auftretende Verschiebung oder Komprimierung von Gewebe bewirkt oder begünstigt werden.

Eine sich beim Einstich biegende Lanzette, die an ihrer Unterseite eine Vertiefung zur Probenaufnahme aufweist, ermöglicht deshalb eine verbesserte Probenaufnahme. Ein Aspekt der Erfindung, der auch selbstständige Bedeutung haben kann, betrifft deshalb eine Lanzette mit einem flachen Schaft der eine Oberseite und eine Unterseite hat, wobei der Schaft an einem vorderen Ende eine Schneide ausbildet, die in einer Spitze endet, die Schneide an der Unterseite zwei Schneidkanten aufweist, die in der Spitze zusammenlaufen, und der Schaft auf seiner Unterseite wenigstens eine Vertiefung zur Probenaufnahme aufweist, wobei der Schaft eine Biegesteifigkeit von weniger als weniger als 0,1 kNmm² (Kilonewton mal Quadratmillimeter), bevorzugt weniger als 0,05 kNmm², insbesondere 0,03 bis 0,001 kNmm²aufweist.

Die Biegesteifigkeit ist das Produkt aus dem Elastizitätsmodul des Materials und dem Flächenträgheitsmoment des Lanzettenschafts. Bei einem Schaft mit rechteckigem Querschnitt beträgt das Flächenträgheitsmoment I=a3^{b}/12, wobei a die Schaftdicke und b die Schaftbreite sind. Bei einer Flachlanzette mit rechteckigem Querschnitt ist das Flächenträgheitsmoment und damit die Biegesteifigkeit wegen der Rinne etwas reduziert.

Bevorzugt ist der Schaft aus Metall, insbesondere Stahl. Als Alternative kann aber auch Kunststoff verwendet werden. Bevorzugt hat der Schaft eine Breite von weniger als 0,5 mm, beispielsweise zwischen 0,2 mm und 0,4 mm. Die Dicke des Schafts beträgt bevorzugt höchstens 0,3mm, besonders bevorzugt zwischen 0,2 mm und 0,05 mm, insbesondere zwischen 0,20 mm und 0,08 mm.

Weitere Einzelheiten und Vorteile der Erfindung werden an Ausführungsbeispielen unter Bezugnahme auf die beigefügten Zeichnungen erläutert. Gleiche und einander entsprechende Teile sind dabei mit übereinstimmenden Bezugszahlen gekennzeichnet. Es zeigen:
- Fig. 1: ein Ausführungsbeispiel einer erfindungsgemäßen Lanzette von unten;
- Fig. 2: eine Seitenansicht zu Figur 1;
- Fig. 3: die in Figur 1 gezeigte Lanzette von oben;
- Fig. 4: eine Schrägansicht der Lanzette;
- Fig. 5: einen Querschnitt entlang der Schnittlinie AA von Figur 3;
- Fig. 6: einen Querschnitt entlang der Schnittlinie BB von Figur 3;
- Fig. 7: einen Querschnitt entlang der Schnittlinie CC von Figur 3;
- Fig. 8: einen Querschnitt entlang der Schnittlinie DD von Figur 3;
- Fig. 9: einen Querschnitt entlang der Schnittlinie EE von Figur 3;
- Fig. 10: einen Querschnitt entlang der Schnittlinie FF von Figur 3;
- Fig. 11: einen Querschnitt entlang der Schnittlinie GG von Figur 3;
- Fig. 12: ein Ausführungsbeispiel einer erfindungsgemäßen Lanzette beim Durchstechen einer Sterilschutzfolie;
- Fig.13: ein Ausführungsbeispiel einer erfindungsgemäßen Lanzette auf einem Trägerband;
- Fig. 14: ein weiteres Ausführungsbeispiel einer erfindungsgemäßen Lanzette;
- Fig. 15: eine Schnittansicht zur Figur 14;
- Fig. 16: ein weiteres Ausführungsbeispiel einer erfindungsgemäßen Lanzette;
- Fig. 17: eine Schnittansicht zur Figur 16;
- Fig. 18: ein weiteres Ausführungsbeispiel einer erfindungsgemäßen Lanzette; und
- Fig. 19: eine Schnittansicht zur Figur 18.

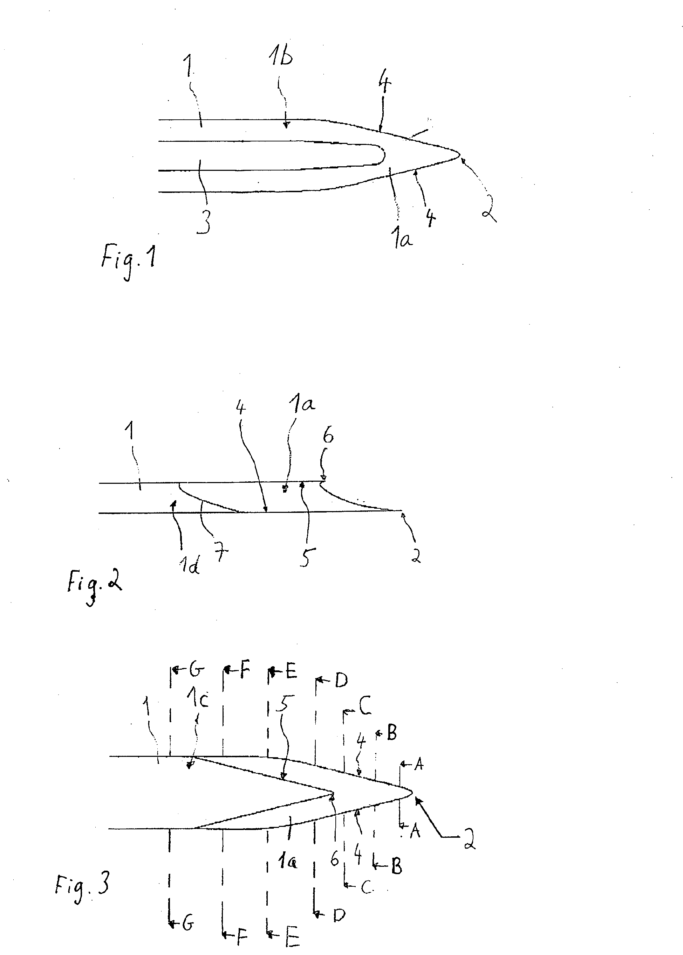

Die in den Figuren 1 bis 11 schematisch dargestellte Lanzette hat einen flachen Schaft 1, der an seinem vorderen Ende eine Schneide 1a ausbildet, die in einer Spitze 2 endet. Der Schaft 1 hat eine in Figur 1 gezeigte Unterseite 1b und eine in Figur 3 gezeigte Oberseite 1 c. Die Unterseite 1b und die Oberseite 1 c sind über sich in Längsrichtung des Schafts 1 erstreckende Schmalseiten 1d miteinander verbunden.

Der Schaft 1 weist auf seiner Unterseite 1b eine Vertiefung 3 zur Probenaufnahme auf. Diese Vertiefung 3 ist bevorzugt als eine Rinne ausgebildet. Es können jedoch auch mehrere Vertiefungen verwendet werden, die beispielsweise als Sacklöcher an der Unterseite 2 angeordnet sind.

Die Schneide 1 a hat zwei untere Kanten, die in der Spitze 2 zusammenlaufen. Diese unteren Kanten sind als Schneidkanten 4 ausgebildet. Bei dem dargestellten Ausführungsbeispiel laufen die Schneidkanten 4 keilförmig zusammen und schließen einen spitzen Winkel ein. Bevorzugt schließen die Schneidkanten 4 einen Winkel von weniger als 60°, bevorzugt von weniger als 45°; insbesondere einen Winkel von weniger als 40° ein. Der zwischen den beiden Schneidkanten 4 eingeschlossene Winkel beträgt bevorzugt mindestens 20°; besonders bevorzugt mindestens 25°.

Zusätzlich zu den beiden Schneidkanten 4 hat die Schneide 1a zwei obere Kanten 5, die in einem Scheitelpunkt 6 zusammenlaufen. Die Schneide 1a ist deshalb an der Oberseite 1 c durch die beiden keilförmig zusammenlaufenden oberen Kanten 5 und an der Unterseite 1b durch die keilförmig zusammenlaufenden Schneidkanten 4 begrenzt. Die oberen Kanten 5 und die Schneidkanten 4 verlaufen bei dem dargestellten Ausführungsbeispiel parallel, können jedoch auch unterschiedliche Winkel einschließen. Bevorzugt schließen die oberen Kanten 5 einen spitzen Winkel ein. Dieser Winkel beträgt bei dem dargestellten Ausführungsbeispiel weniger als 60°, beispielsweise 10° bis 50°, insbesondere 20° bis 45°.

Der Scheitelpunkt 6 ist bevorzugt um mehr als die Dicke des Schaftes 1 hinter der Spitze 2 angeordnet. Die in Längsrichtung des Schaftes 1 gemessene Komponente des Abstandes zwischen dem Scheitelpunkt 6 und der Spitze 2, d.h. dem vorderen Endpunkt der Lanzette, beträgt also vorzugsweise mehr als die Dicke des Schaftes 1. Besonders bevorzugt ist der Scheitelpunkt 6, wie bei dem dargestellten Ausführungsbeispiel um mehr als das Doppelte der Dicke des Schaftes 1 hinter der Spitze 2 angeordnet. Bevorzugt beträgt die Breite des Schaftes 1 das zwei- bis dreifache seiner Dicke.

Die oberen Kanten 5 erstrecken sich weiter nach hinten als die Schneidkanten 4. Die Schneide 1a ist seitlich auf beiden Seiten jeweils durch eine Kante 7 begrenzt, die das hintere Ende einer Schneidkante 4 mit dem hinteren Ende einer oberen Kante 5 verbindet. Die Kante 7 schließt mit der Unterkante der auf ihrer Seite angeordneten Schmalseite 1d bevorzugt einen spitzen Winkel ein, beispielsweise eine Winkel zwischen 10° und 60°, insbesondere zwischen 15° und 35°. Mit der auf ihrer Seite angeordneten Schneidkante 4 kann die Kante 7 einen stumpfen Winkel einschließen.

In Figur 2 ist dabei zu erkennen, dass die Schneide 1 a entlang einer von dem Scheitelpunkt 6 zu dem vorderen Ende 2 verlaufenden Linie konkav geformt ist. Die Oberseite 1 b ist an dem Scheitelpunkt 6 hinterschnitten.

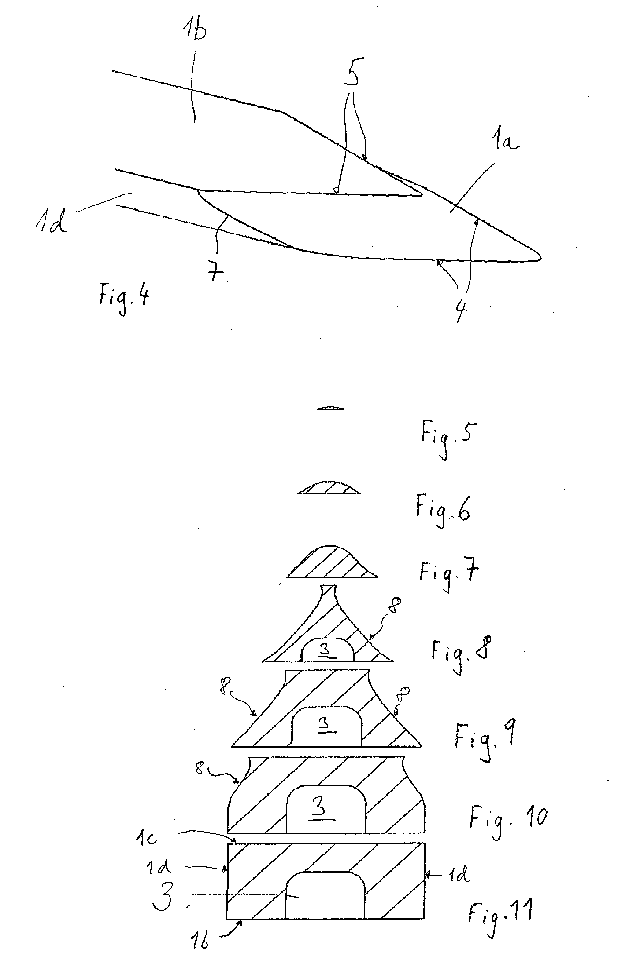

Die Figuren 5 bis 11 zeigen eine Serie von Querschnitten der Lanzette entlang der in Figur 3 dargestellten Schnittlinien. Dabei zeigt Figur 5 eine Schnittansicht entlang der Schnittlinie AA, Figur 6 entlang der Schnittlinie BB, Figur 7 entlang der Schnittlinie CC, Figur 8 entlang der Schnittlinie DD, Figur 9 entlang der Schnittlinie EE, Figur 10 entlang der Schnittlinie FF und Figur 11 entlang der Schnittlinie GG.

In den Figuren 5 bis 7 ist zu erkennen, dass die Oberseite der Schneide 1a zwischen dem Scheitelpunkt 6 und dem vorderen Endpunkt 2 im Querschnitt gesehen konvex gewölbt ist. Die Figuren 5 bis 7 zeigen dabei auch, dass die Schneidkanten 4 einen zum vorderen Ende 2 hin abnehmenden Schneidwinkel aufweisen. Der Schneidwinkel nimmt dabei kontinuierlich zum vorderen Ende 2 hin ab. Dies führt dazu, dass der bei einem Lanzettenstich im Körpergewebe eines Patienten erzeugte Einstichkanal kontinuierlich dicker und breiter wird, was für einen schmerzarmen Einstich vorteilhaft ist.

Die Figuren 8 bis 10 zeigen, dass die Schneide 1a hinter dem Scheitelpunkt 6, insbesondere zwischen dem Scheitelpunkt 6 und dem hinteren Ende der Schneidkanten 4, konkav geformte Seitenflächen 8 hat

Die Unterseite 1b des Schafts 1 ist plan oder konkav. Die Figuren 5 bis 11 zeigen dabei, dass die Unterseite 1b des Schafts 1, insbesondere die Unterseite der Schneide 1 a, beidseitig in einem Randbereich plan ist. Abgesehen von der Vertiefung 3 zur Probenaufnahme ist die gesamte Unterseite 1b des Schafts 1 plan. Die Vertiefung 3 führt dazu, dass die Unterseite 1 b des Schafts 1 in dem entsprechenden Bereich konkav ist.

Bei einer erfindungsgemäßen Lanzette kann die Unterseite 1 b zwischen dem vorderen Ende 2 und dem Scheitel 6 oder sogar die gesamte Unterseite der Schneide 1 a frei von Vertiefungen 3 zur Probenaufnahme sein. Bevorzugt erstreckt sich die Vertiefung 3 aber auch im Bereich der Schneide 1a. Um die für eine Probengewinnung erforderliche Einstichtiefe zu minimieren, ist es in der Regel vorteilhaft, wenn sich die Vertiefung 3 an der Unterseite 1b in den Bereich zwischen den Scheitelpunkt 6 und dem vorderen Ende 2 hineinerstreckt. Um die mechanische Stabilität der Schneide 1a nicht zu beeinträchtigen, ist es im Allgemeinen vorteilhaft, wenn die Rinne 3 in einem Abstand von dem vorderen Ende 2 endet. Vorteilhaft an einer solchen an der Unterseite 1b des Schafts angeordneten Rinne 3 ist, dass sie beim Durchstechen eines Sterilschutzes nicht durch Material des Sterilschutzes verstopft wird.

Bei dem dargestellten Ausführungsbeispiel erstreckt sich die Rinne 3 über den Scheitelpunkt 6 hinaus. In Figur 1 ist dabei zu erkennen, dass sich die Rinne 3 in einem Abschnitt im Bereich der Schneide 1a kontinuierlich verjüngt. Die Länge des sich verjüngenden Abschnitts ist dabei größer als die maximale Breite der Rinne 3. In dem gesamten sich verjüngenden Abschnitt nimmt die Tiefe der Rinne 3 zum vorderen Ende hin kontinuierlich ab. Die Querschnittsfläche der Rinne 3 nimmt also in dem zwischen den Schneidkanten 4 angeordneten Abschnitt ab.

Die maximale Breite der Rinne 3, also die Rinnenbreite hinter der Schneide 1 a, beträgt bevorzugt weniger als die Hälfte der Schaftbreite. Die maximale Tiefe der Rinne, also die Rinnentiefe hinter der Schneide 1 a, beträgt bevorzugt mehr als die Hälfte der Schaftdicke. Die Schaftdicke sollte weniger als 0,3 mm betragen und liegt bevorzugt zwischen 80 µm und 200 µm, besonders bevorzugt zwischen 80 µm und 180 µm. Die Schaftbreite kann beispielsweise zwischen 0,2 mm und 0,5 mm, bevorzugt 250 µm bis 400 µm betragen.

Als Material ist Stahl, insbesondere Edelstahl, bevorzugt. Die Biegesteifigkeit des Lanzettenschafts sollte 0,1 kNmm² nicht übersteigen, bevorzugt weniger als 0,05 kNmm², besonders bevorzugt weniger als 0,02 kNmm² betragen. Vorteilhaft sind insbesondere Werte von 0,01 bis 0,001 kNmm².

Lanzetten mit einer derart geringen Biegesteifigkeit werden beim Einstich in Körpergewebe elastisch gebogen. Die die Rinne 3 aufweisende Unterseite der Lanzette ist dabei die Innenseite der Biegung, so dass beim Zurückziehen der Lanzette an der Unterseite und damit im Bereich der Rinne 3 ein mit Körperflüssigkeit gefüllter Hohlraum entsteht. Durch die Biegung der Lanzette wird deshalb die Probenaufnahme verbessert.

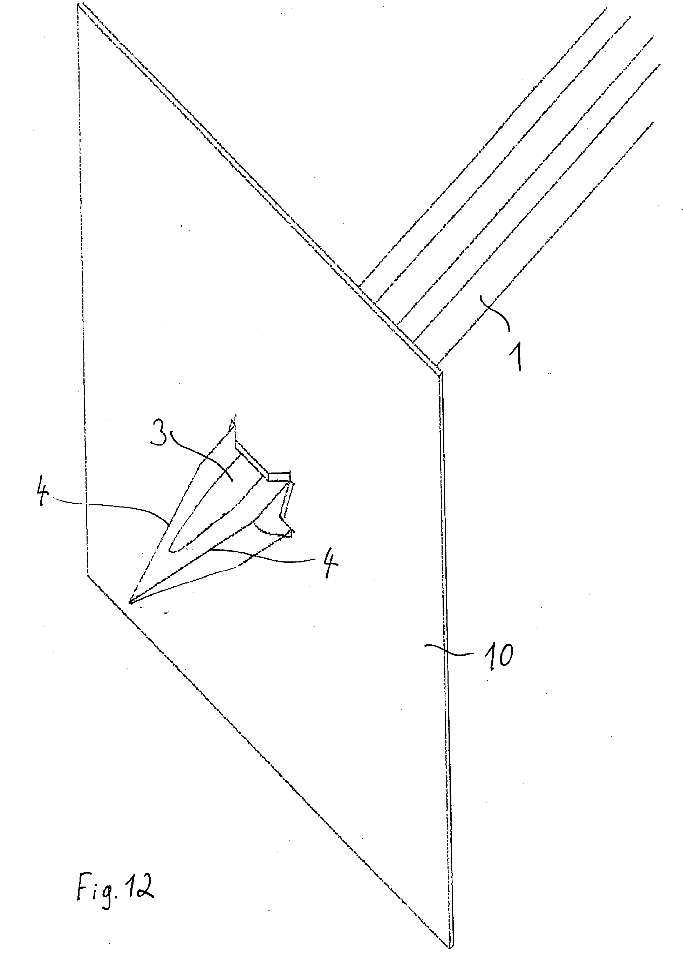

In Figur 12 ist ein Ausführungsbeispiel einer erfindungsgemäßen Lanzette beim Durchstechen einer Sterilschutzfolie 10, die beispielsweise eine Metallfolie, insbesondere eine Aluminiumfolie, eine Kunststofffolie oder eine mit Kunststoff beschichtete Metallfolie bzw. eine Metall beschichtet Kunststofffolie sein kann, dargestellt. Mit einer solchen Sterilschutzfolie 10 kann beispielsweise eine Kammer eines Lanzettenmagazins verschlossen sein, um eine darin angeordnete Lanzette vor schädlichen Umwelteinflüssen zu schützen. Da bei einer erfindungsgemäßen Lanzette die Rinne 3 zur Probenaufnahme an der Unterseite des Schafts angeordnet ist, wird die Rinne 3 beim Durchstechen der Sterilschutzfolie 10 nicht beeinträchtigt. Die Schneidkanten 4 der Lanzette bewirken nämlich, dass die Sterilschutzfolie 10 an der Unterseite der Lanzette parallel zur Schaftunterseite aufgeschnitten wird, so dass dem Entstehen von Folienresten, welche die Rinne 3 verstopfen könnten, entgegengewirkt wird. Vorteilhaft wird auch vermieden, das Teile der durchstochenen Sterilschutzfolie 10 beim Durchstoßen in das innere der Rinne 3 hineinragen und durch Entlanggleiten an einer Oberfläche der Rinne 3 eine dort bevorzugt vorhandene hydrophile Beschichtung beeinträchtigen.

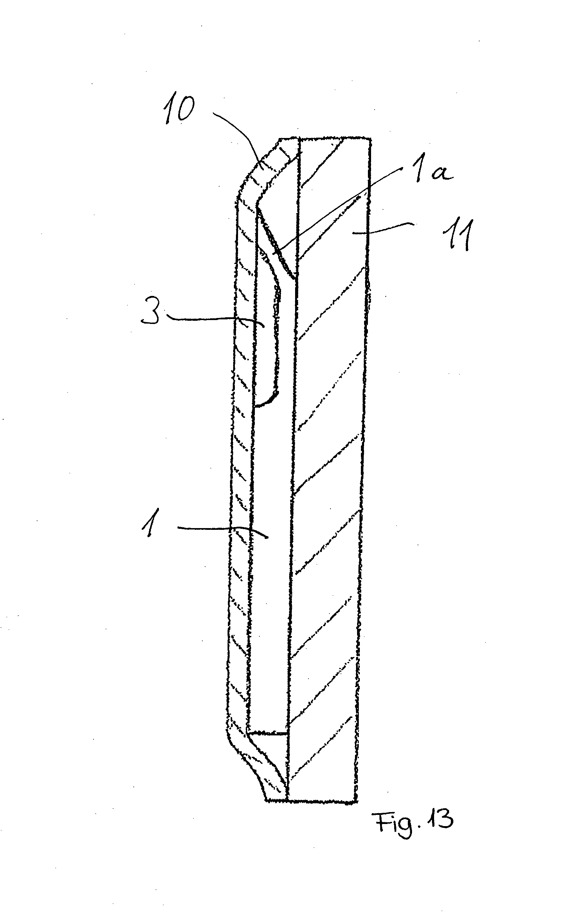

Anstatt eine erfindungsgemäße Lanzette in einer mit Sterilschutzfolie 10 verschlossenen Magazinkammer anzuordnen, kann man erfindungsgemäße Lanzetten auch nebeneinander auf einem Trägerband anordnen, wie dies beispielsweise aus der WO 2008/083844 A1 bekannt ist. Bevorzugt wird eine erfindungsgemäße Lanzette mit ihrer Oberseite auf einem Trägerband angeordnet und deren Unterseite von einer Sterilschutzfolie bedeckt. Figur 13 zeigt ein entsprechendes Ausführungsbeispiel mit einem Trägerband 11, einer Lanzette und einer Sterilschutzfolie 10 in einer Schnittansicht.

Die Sterilschutzfolie 10 ist dünner als das Trägerband 11, bevorzugt höchstens halb so dick. Die Sterilschutzfolie 10 liegt lose auf der Lanzette auf und ist mit dem Trägerband 11 stoffschlüssig verbunden, beispielsweise verklebt oder verschweißt. Der Lanzettenschaft 1 kann in einem hinteren, von der Schneide 1a entfernten Bereich mit dem Trägerband 11 verklebt sein. In einem vorderen Bereich liegt der Lanzetteschaft 1 bevorzugt lose auf dem Trägerband 11 auf, so dass die Schneide 1a für einen Stich durch Biegen des Trägerbandes 11 von der Sterilschutzfolie befreit werden kann, wie dies in der WO 2008/083844 A1 beschrieben ist.

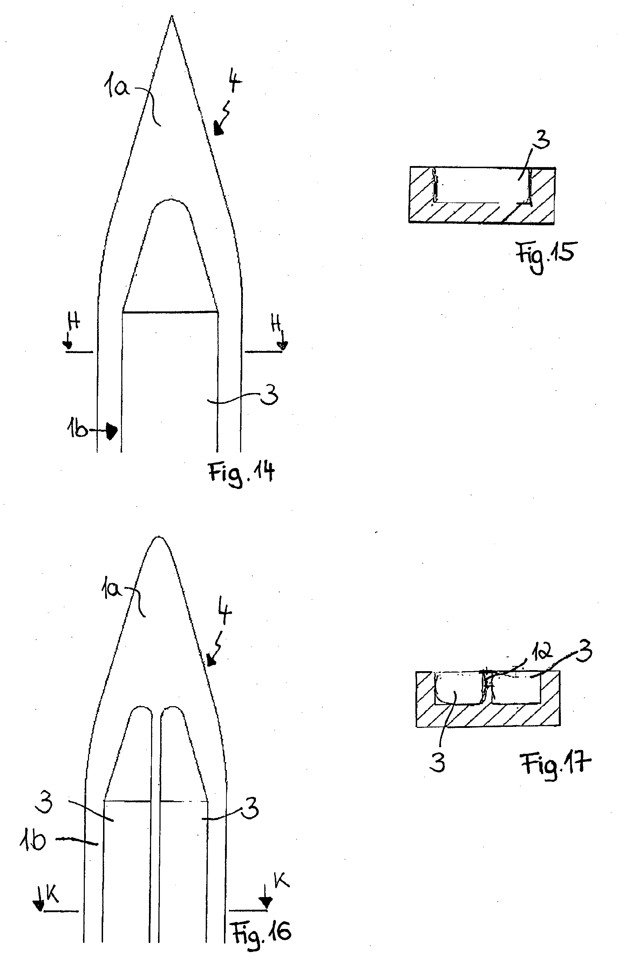

Figur 14 zeigt ein weiteres Ausführungsbeispiel einer Lanzette und Fig. 15 eine dazugehörende Schnittansicht entlang der Schnittlinie HH. Diese Lanzette unterscheidet sich von der in den Figuren 1 bis 11 dargstellten Lanzette nur dadurch, dass die Vertiefung 3 zur Probenaufnahme als eine etwas breitere Rinne ausgebildet ist. Dies ermöglicht die Aufnahme von größeren Probenvolumen.

Figur 16 zeigt ein weiteres Ausführungsbeispiel einer Lanzette und Fig. 17 eine dazugehörende Schnittansicht entlang der Schnittlinie KK. Diese Lanzette unterscheidet sich von dem Ausführungsbeispiel der Figuren 14 und 15 dadurch, dass anstelle einer einzigen als Rinne ausgebildeten Vertiefung 3 zwei parallel verlaufende als Rinne ausgebildete Vertiefungen 3 vorhanden sind. Die Trennwand 12 zwischen den beiden Vertiefungen 3 reduziert das Gesamtvolumen der beiden Vertiefungen 3 nur unwesentlich, führt jedoch zu wesentlich größeren Kapillarkräften. Die beiden Vertiefungen 3 des in Figur 16 dargestellten Ausführungsbeispiels füllen sich deshalb schneller mit Körperflüssigkeit als die eine Vertiefung des in den Figuren 14 und 15 dargestellten Ausführungsbeispiels.

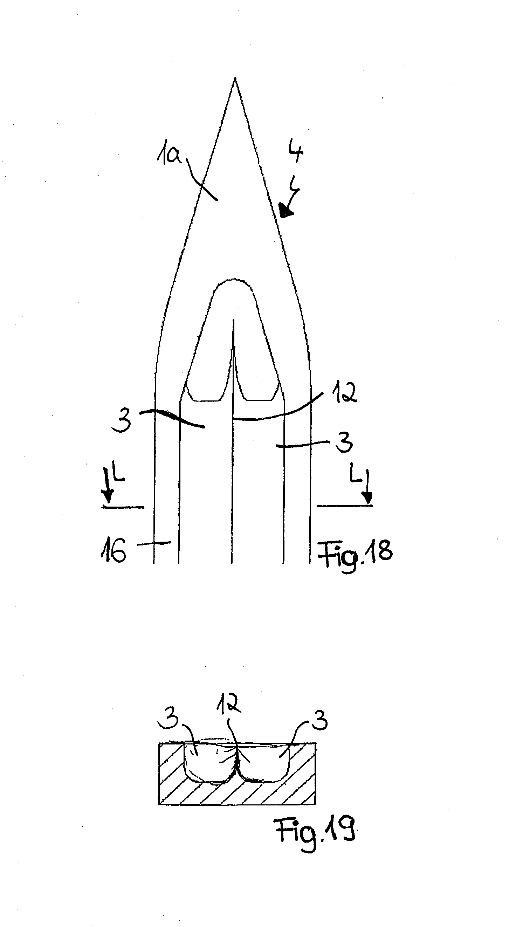

Figur 18 zeigt ein weiteres Ausführungsbeispiel einer Lanzette und Fig. 19 eine dazugehörende Schnittansicht entlang der Schnittlinie LL. Diese Lanzette unterscheidet sich von dem Ausführungsbeispiel der Figur 18 nur dadurch, dass die Trennwand 12 zwischen den beiden Vertiefungen 3 reduziert ist. Die beiden Vertiefungen 3 sind deshalb an der Oberfläche verbunden, im Inneren jedoch durch Trennwand 12 in zwei parallele Kanäle unterteilt. Die Trennwand 12 führt ebenfalls zu einer Erhöhung der Kapillarkräfte und somit zu einem schnelleren Füllen der Vertiefung 3.

### Bezugszahlen

- 1: Schaft
- 1a: Schneide
- 1b: Unterseite
- 1c: Oberseite
- 1d: Schmalseite
- 2: Spitze
- 3: Vertiefung
- 4: Schneidkante
- 5: obere Kante
- 6: Scheitelpunkt
- 7: seitliche Begrenzungskante
- 8: Seitenflächen
- 10: Sterilschutzfolie
- 11: Trägerband
- 12: Trennwand

## Patentansprüche

1. Lanzette mit einem flachen Schaft (1), der eine Oberseite (1c) und eine Unterseite (1 b) hat, wobei
der Schaft (1) an einem vorderen Ende (2) eine Schneide (1a) ausbildet, die in einer Spitze (2) endet,
die Schneide (1a) an der Unterseite (1b) zwei Schneidkanten (4) aufweist, die in der Spitze (2) zusammenlaufen, und
der Schaft (1) auf seiner Unterseite (1 b) wenigstens eine Rinne (3) zur Probenaufnahme aufweist, **dadurch gekennzeichnet, dass**
die Rinne (3) einen zwischen den Schneidkanten (4) angeordneten Abschnitt aufweist, in welchem die Querschnittsfläche der Rinne (3) zu dem vorderen Ende (2) hin abnimmt, und
der Schaft an seiner Oberseite (1 c) an zwei Kanten (5), die in einem Scheitelpunkt (6) zusammenlaufen, in die Schneide (1a) übergeht, und sich die Rinne (3) über den Scheitelpunkt (6) hinaus erstreckt.

2. Lanzette nach Anspruch 1, **dadurch gekennzeichnet, dass** die Schneide (1 a) am Ende der Rinne (3) weniger als zwei Drittel der Dicke des Schafts hat.

3. Lanzette nach einem der vorstehenden Ansprüche, **dadurch gekennzeichnet, dass** sich die Tiefe der Rinne (3) im Bereich der Schneide (1a) reduziert.

4. Lanzette nach Anspruch 3, **dadurch gekennzeichnet, dass** die Tiefe der Rinne (3) auf einer Länge abnimmt, die größer als die maximale Breite der Rinne (3) ist.

5. Lanzette nach einem der vorstehenden Ansprüche, **dadurch gekennzeichnet, dass** die Breite der Rinne (3) im Bereich der Schneide (1a) reduziert.

6. Lanzette nach einem der vorstehenden Ansprüche, **dadurch gekennzeichnet, dass** die Querschnittsfläche der Rinne (3) auf einer Länge abnimmt, die größer als die Schaftdicke, vorzugsweise größer als die Schaftbreite ist.

7. Lanzette nach einem der vorstehenden Ansprüche, **dadurch gekennzeichnet, dass** sich die Rinne (3) im Bereich der Schneide (1a) kontinuierlich verjüngt.

8. Lanzette nach einem der vorstehenden Ansprüche, **dadurch gekennzeichnet, dass** die Schneide (1a) entlang einer von dem Scheitelpunkt (6) zu dem vorderen Ende (2) verlaufenden Linie konkav geformt ist.

9. Lanzette nach einem der vorstehenden Ansprüche, **dadurch gekennzeichnet, dass** die Oberseite (1c) an dem Scheitelpunkt (6) einen Hinterschnitt aufweist.

10. Lanzette nach einem der vorstehenden Ansprüche, **dadurch gekennzeichnet, dass** die Schneide (1a) zwischen dem Scheitelpunkt (6) und dem hinteren Ende der Schneidkanten (4) konkav geformte Seitenflächen (8) hat.

11. Lanzette nach einem der vorstehenden Ansprüche, **dadurch gekennzeichnet, dass** die Schneidkanten (4) einen zu dem vorderen Ende (2) hin abnehmenden Schneidwinkel aufweisen.

12. Lanzette nach einem der vorstehenden Ansprüche, **dadurch gekennzeichnet, dass** die Unterseite (1 b) der Schneide (1a) zumindest in einem entlang der Schneidkanten (4) verlaufenden Randbereich plan ist.

13. Lanzette nach einem der vorstehenden Ansprüche, **dadurch gekennzeichnet, dass** sich die oberen Kanten (5) weiter nach hinten als die Schneidkanten (4) erstrecken.

14. Stechsystem mit einer Lanzette nach einem der vorstehenden Ansprüche und einem Stechgerät, das bei einem Stich eine Vorschubbewegung der Lanzette und eine daran anschließende Rückführbewegung bewirkt, wobei die Rückführbewegung langsamer als die Vorschubbewegung ist.

## Claims

1. Lancet comprising a flat shank (1) having a top side (1c) and a bottom side (1b), the shank (1) forming a blade (1a) at a forward end (2), the blade (1a) terminating in a point (2),
the blade (1a) having two cutting edges (4) at the bottom side (1b), the cutting edges (4) converging in the point (2), and
the shank (1) having on its bottom side (1b) at least one groove (3) for receiving a sample,
**characterized in that**
between the cutting edges (4) the groove (3) has a section wherein the cross-sectional area of the groove (3) decreases towards the forward end (2), and
the shank turns into the blade (1a) on its top side (1c) at two edges (5) which converge in a vertex (6), and
that the groove extends beyond the vertex (6).

2. Lancet according to claim 1, **characterized in that** blade (1a) has at the end of the groove (3) a thickness that is less than two thirds of the thickness of the shank.

3. Lancet according to any one of the preceding claims, **characterized in that** the depth of the groove (3) decreases in the region of the blade (1a).

4. Lancet according to claim 3, **characterized in that** the depth of the groove (3) decreases along a length that is larger than the maximum width of the groove (3).

5. Lancet according any one of the preceding claims, **characterized in that** the width of the groove (3) is reduced in the region of the blade (1a). -

6. Lancet according any one of the preceding claims, **characterized in that** the cross-sectional area of the groove (3) decreases along a length that is larger than the thickness of the shaft, preferably larger than the width of the shaft.

7. Lancet according any one of the preceding claims, **characterized in that** groove (3) tapers continuously in the region of the blade (1a) .

8. Lancet according any one of the preceding claims, **characterized in that** the blade (1a) is formed concavely along a line running from the vertex (6) to the forward end (2).

9. Lancet according to any one of the preceding claims, **characterized in that** the top side (1c) has an undercut at the vertex (6).

10. Lancet according to any one of the preceding claims, **characterized in that** the blade (1a) has concave lateral surfaces (8) between the vertex (6) and the rear end of the cutting edges (4).

11. Lancet according to any one of the preceding claims, **characterized in that** the cutting edges (4) have a cutting angle that decreases towards the forward end (2).

12. Lancet according to any one of the preceding claims, **characterized in that** the bottom side (1b) of the blade (1a) is plane at least in a marginal region along the cutting edges (4).

13. Lancet according to any one of the preceding claims, **characterized in that** the upper edges (5) extend further back than the cutting edges (4).

14. Puncturing system comprising a lancet according to any one of the preceding claims and a puncturing device that, during a puncture, causes a forward motion of the lancet and subsequently a retracting motion, wherein the retracting motion is slower than the forward motion.

## Revendications

1. Lancette avec une tige plate (1), qui a une face supérieure (1 c) et une face inférieure (1 b), dans laquelle
la tige (1) forme au niveau d'une extrémité avant (2) une arête vive (1a), qui se termine par une pointe (2),
l'arête vive (1a) présente au niveau de la face inférieure (1 b) deux bords coupants (4), qui convergent dans la pointe (2), et
la tige (1) présente sur sa face inférieure (1b) au moins un conduit (3) pour le recueil d'échantillons, **caractérisée en ce que**
le conduit (3) présente une section disposée entre les bords coupants (4), dans laquelle l'aire de la section transversale du conduit (3) diminue dans la direction de l'extrémité avant (2), et
l'arbre au niveau de sa face supérieure (1 c) au niveau de deux bords (5), qui convergent dans un sommet (6), passe dans l'arête vive (1a), et le conduit (3) s'étend au-delà du sommet (6).

2. Lancette selon la revendication 1, **caractérisée en ce que** l'arête vive (1a) a à l'extrémité du conduit (3) moins des deux tiers de l'épaisseur de la tige.

3. Lancette selon l'une des revendications précédentes, **caractérisée en ce que** la profondeur du conduit (3) se réduit dans la zone de l'arête vive (1 a).

4. Lancette selon la revendication 3, **caractérisée en ce que** la profondeur du conduit (3) diminue jusqu'à une longueur qui est supérieure à la largeur maximale du conduit (3).

5. Lancette selon l'une des revendications précédentes, **caractérisée en ce que** la largeur du conduit (3) réduit dans la zone de l'arête vive (1a).

6. Lancette selon l'une des revendications précédentes, **caractérisée en ce que** l'aire de la section transversale du conduit (3) réduit jusqu'à une longueur qui est supérieure à l'épaisseur de la tige, de préférence supérieure à la largeur de la tige.

7. Lancette selon l'une des revendications précédentes, **caractérisée en ce que** le conduit (3) rétrécit de manière continue dans la zone de l'arête vive (1a).

8. Lancette selon l'une des revendications précédentes, **caractérisée en ce que** l'arête vive (1a) est formée de manière concave le long d'une ligne allant du sommet (6) à l'extrémité avant (2).

9. Lancette selon l'une des revendications précédentes, **caractérisée en ce que** la face supérieure (1 c) présente au niveau du sommet (6) une contre-dépouille.

10. Lancette selon l'une des revendications précédentes, **caractérisée en ce que** l'arête vive (1a) a entre le sommet (6) et l'extrémité arrière des bords coupants (4) des surfaces latérales formées de manière concave (8).

11. Lancette selon l'une des revendications précédentes, **caractérisée en ce que** les bords coupants (4) présentent un angle coupant qui se rétrécit dans la direction de l'extrémité avant (2).

12. Lancette selon l'une des revendications précédentes, **caractérisée en ce que** la face inférieure (1b) de l'arête vive (1a) est plane au moins dans une zone de bord s'étendant le long des bords coupants (4).

13. Lancette selon l'une des revendications précédentes, **caractérisée en ce que** les bords supérieurs (5) s'étendent vers l'arrière plus loin que les bords coupants (4).

14. Système de piqûre avec une lancette selon l'une des revendications précédentes et un appareil autopiqueur, qui exerce lors d'une piqûre un mouvement de poussée de la lancette et un mouvement de rétroaction qui s'ensuit, dans lequel le mouvement de rétroaction est plus lent que le mouvement de poussée.
